# EUROPEAN PATENT APPLICATION

(11) **EP 3 834 918 A1**
(43) Date of publication of application: **16.06.2021**
(21) Application number: 20211247.0
(22) Date of filing: 02.12.2020
(51) Int. Cl.: B01D 53/86, F25D 17/04, A61L 9/22

(54) **GAS PURIFICATION DEVICE, METHOD FOR PURIFICATION USING THE SAME, AND REFRIGERATOR**

(30) Priority: 11.12.2019 CN 201911265755
(71) Applicant: BSH Hausgeräte GmbH, 81739 München (DE)
(72) Inventor: Liu, Yuhua, Nanjing, Jiangsu 210046 (CN); Winner, Sebastian, 58644 Iserlohn (DE); Zhong, Wei, Nanjing, Jiangsu 210046 (CN)

(57) **Abstract**

A gas purification device (100) for a refrigerator, a method for purification through the gas purification device (100), and a refrigerator are provided in the embodiments of this application. The gas purification device (100) includes: an airflow channel, having an air inlet (101) and an air outlet (102); a fan module (110), disposed in the airflow channel, and adapted to direct an airflow from the air inlet (101) to the air outlet (102); an ion generator (120), disposed in the airflow channel, and adapted to generate ions; and a filter (130) including a catalyst, disposed in the airflow channel and downstream of the ion generator (120), where an airflow including the ions flows through the filter (130). The gas purification device (100) provided in the embodiments of this application has remarkably improved decomposition capacity and purification capacity.

## Description

### BACKGROUND

### Technical Field

The present invention relates to the field of gas purification, and particularly, to a gas purification device, a method for purification through the gas purification device, and a refrigerator.

### Related Art

In an enclosed environment, an internal gas usually is recycled. As time passes, pollutants (for example, bacteria and volatile organic compounds (VOC)) in the internal gas gradually increase, and these pollutants come from food, clothes, human bodies, industrial products, or the like.

Various purification methods are provided in the prior art. For example, an activated catalyst can produce a lot of reactive components, so that an airflow containing odor gas molecules such as VOC is decomposed when flowing through the catalyst.

However, the catalyst usually needs to be used at a relative high temperature (for example, a temperature in a range of 20 to 30 degrees), and has a low purification capacity in an enclosed environment at a relatively low temperature, for example, a refrigerator.

### SUMMARY

Embodiments of the present invention are provided to resolve the technical problem of a low purification capacity of a catalyst in an enclosed environment at a relatively low temperature.

To resolve the foregoing technical problem, a gas purification device for a refrigerator is provided in the embodiments of the present invention, including: an airflow channel, having an air inlet and an air outlet; a fan module, disposed in the airflow channel, and adapted to direct an airflow from the air inlet to the air outlet; an ion generator, disposed in the airflow channel, and adapted to generate ions; and a filter including a catalyst, disposed in the airflow channel and downstream of the ion generator, where an airflow including the ions flows through the filter.

Optionally, the fan module is disposed upstream or downstream of the ion generator.

Optionally, the ions have kinetic energy for movement towards the filter.

Optionally, an electrode of the ion generator includes a needle-like portion, and an end of the needle-like portion faces the filter.

Optionally, the gas purification device includes a first electrode and a second electrode, wherein the airflow flows along an extension direction of the first electrode and/or the second electrode in the ion generator.

Optionally, the ion generator includes a passage located between the first electrode and/or the second electrode and a shell of the ion generator, and the airflow flows through the passage.

Optionally, the ions include negative ions.

Optionally, the catalyst includes a metal oxide or a precious metal.

Optionally, the ion generator is adapted to continuously operate for at least 5 minutes.

Optionally, an operating voltage of the ion generator is in a range of -3 KV to -7 KV.

Optionally, a distance between the ion generator and the filter is in a range of 10 mm to 500 mm.

Optionally, the distance between the ion generator and the filter is in a range of 20 mm to 30 mm.

Optionally, the gas purification device includes a housing, wherein the airflow channel, the fan module, the ion generator, and the filter are all located in the housing.

Optionally, the gas purification device is adapted to operate in a first mode or a second mode, wherein in the first mode, the fan module operates but the ion generator does not operate, and in the second mode, the fan module operates and the ion generator operates.

Optionally, the ion generator is adapted to start or stop operating according to an operation of the refrigerator.

Optionally, the gas purification device includes an odor sensor, wherein the ion generator is adapted to determine, according to a concentration value of an odor gas sensed by the odor sensor, whether to operate.

Optionally, the gas purification device is adapted to determine whether the concentration value is higher than a preset threshold; if yes, the ion generator operates; otherwise, the ion generator does not operate.

Optionally, the fan module, the ion generator, and the filter are located in an air duct adapted to deliver cold air to a storage compartment of the refrigerator.

A refrigerator is further provided in the embodiments of the present invention, further including the gas purification device described above.

A method for purification through a gas purification device is further provided in the embodiments of the present invention, the gas purification device including a fan module, an ion generator, and a filter including a catalyst that are disposed in an airflow channel, the filter being disposed downstream of the ion generator, the gas purification device being adapted to operate in a first mode or a second mode, wherein in the first mode, the fan module operates but the ion generator does not operate so that an airflow passes through the filter, and in the second mode, the fan module operates and the ion generator operates so that ions included in an airflow come into contact with the catalyst when the airflow passes through the filter; the method includes: receiving a control signal; and causing, based on the control signal, the gas purification device to operate in the first mode or the second mode.

Optionally, the receiving a control signal includes receiving a control signal generated according to an operation on a refrigerator.

Optionally, the gas purification device includes an odor sensor adapted to sense a concentration value of an odor gas, and the receiving a control signal includes receiving a control signal generated according to the concentration value.

Optionally, ions generated by the ion generator have kinetic energy for movement towards the filter.

Optionally, an electrode of the ion generator includes a needle-like portion, and an end of the needle-like portion faces the filter.

Optionally, the gas purification device is disposed in a storage compartment of the refrigerator.

Compared with the prior art, the technical solutions of the embodiments of the present invention have beneficial effects. For example, high-energy ions generated by the ion generator hit the catalyst in the filter, the catalyst obtains energy after being hit, and the temperature of the catalyst significantly increases. Accordingly, the decomposition capacity and purification capacity of the catalyst are remarkably improved.

### BRIEF DESCRIPTION OF THE DRAWINGS

FIG. 1 is a schematic structural diagram of a gas purification device according to an embodiment of the present invention;
FIG. 2 is a schematic diagram of a position relationship between an ion generator and a filter including a catalyst according to an embodiment of the present invention;
FIG. 3 is a schematic structural diagram of a gas purification device located in an air duct according to an embodiment of the present invention;
FIG. 4 is a schematic structural diagram of a refrigerator including a gas purification device according to an embodiment of the present invention; and
FIG. 5 is a flowchart of a method for purification through a gas purification device according to an embodiment of the present invention.

### DETAILED DESCRIPTION

To make the objectives, features, and beneficial effects of the embodiments of the present invention more comprehensible, the following describes the specific embodiments of the present invention in detail with reference to the accompanying drawings.

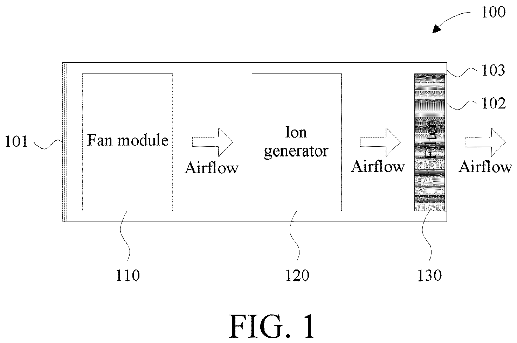

As shown in FIG. 1, a gas purification device 100 includes an airflow channel, a fan module 110, an ion generator 120, and a filter 130 including a catalyst. The airflow through the airflow channel is indicated by an arrow direction in FIG. 1. Directed by the fan module 110, an airflow can flow along the airflow channel from an air inlet 101 to an air outlet 102. Accordingly, the fan module 110 is located in the airflow channel and upstream or downstream of the ion generator 120. In this embodiment of the present invention, the downstream and upstream are opposite positions defined relative to the flow direction of the airflow in the airflow channel. The airflow flows from the upstream to the downstream. The fan module 110 directs a to-be-purified gas to the filter 130 to sterilize and remove odors from the to-be-purified gas in the filter 130, and directs ions, ozone, and the like generated by the ion generator 120 to the filter 130. In the filter 130, the ions can not only activate the catalyst in the filter 130, but also remove odors from the gas, and the ozone can sterilize and remove odors from the gas.

The ion generator 120 is disposed in the airflow channel, and is adapted to produce ions, ozone, and the like. The ion generator 120 can continuously operate. A shortest time for continuous operation, for example, 5 minutes, can be set according to an operating cost, activation efficiency, effects of sterilization and odor removal, and the like.

The ion generator 120 may start or stop operating according to an operation on a refrigerator. For example, the ion generator 120 starts operating according to a received operation signal of closing a refrigerator door, stops operating according to a received operation signal of opening the refrigerator door, starts or stops operating according to a received signal of a refrigerating system, and starts or stops operating according to a received signal inputted by a user. The gas purification device 100 may include an odor sensor, and can further cause, according to a concentration value of an odor gas (for example, VOC) sensed by the odor sensor, the ion generator 120 to start or stop operating. In specific implementation, a preset threshold of the concentration of the odor gas can be set. If the sensed concentration value is higher than the preset threshold, the ion generator 120 starts operating; otherwise, the ion generator 120 does not operate. The preset threshold is in a range of 500 ppm to 1500 ppm. Accordingly, the gas purification device 100 may operate in a first mode or a second mode. In the first mode, the fan module 110 operates and the ion generator 120 does not operate, and in the second mode, the fan module 110 operates and the ion generator 120 operates.

A distance between the ion generator 120 and the filter 130 needs to be set. If the distance is relatively long, the energy of the irons is relatively low when the irons reach the catalyst in the filter 130, resulting in a relatively weak catalyst activation effect. If the distance is relatively short, the ions generated by the ion generator 120 gather in some regions of the catalyst, and therefore the size utilization of the catalyst is relatively low. In specific implementation, the distance between the ion generator 120 and the filter 130 is set in a range of 10 mm to 500 mm. In a preferred embodiment, the distance is set in a range of 20 mm to 30 mm.

The filter 130 including the catalyst is disposed in the airflow channel, and is located downstream of the ion generator 120. The airflow includes the ions generated by the ion generator 120 and flows through the filter 130. The filter 130 including the catalyst is a filtration structure with the catalyst disposed therein. The filtration structure has a conventional structure in this field and is used for filtering odor molecules and removing odors. The catalyst includes a metal oxide or a precious metal. The metal oxide may be silver oxide, copper oxide, cerium oxide, nickel oxide, cobaltous oxide, lanthanum oxide, zirconium oxide, manganese oxide, or the like. The precious metal may be gold, rhodium, platinum, palladium, or the like. The catalyst can be fully activated only at a relatively high temperature (for example, a temperature in a range of 20 to 30 degrees), to produce a lot of reactive components to decompose odor molecules and purify gas. Therefore, it is challenging to apply the catalyst in a low-temperature environment (for example, a refrigeration or freezing environment of a refrigerator).

The ions generated by the ion generator 120 have kinetic energy for movement towards the filter 130. The kinetic energy causes the ions to hit the catalyst in the filter 130, the catalyst obtains energy after being hit, and the temperature of the catalyst significantly increases; accordingly, the decomposition capacity and purification capacity of the catalyst are remarkably improved. When the ion generator is used, in the prior art, it is only considered that the ion generator can generate ions having an odor removal function. However, it is not considered in the prior art that the kinetic energy of the ions can further be used for activating the catalyst so that the temperature of the catalyst increases significantly; therefore most of the odor gas molecules in the airflow, for example, VOC, are catalyzed and decomposed when the odor gas molecules pass through the catalyst, and some ozone in the airflow is also catalyzed and decomposed, thereby reducing the concentration of the ozone, avoiding strong irritating odors produced inside the refrigerator by the ozone, and also avoiding oxidation of inner walls of the refrigerator by the high-concentration ozone. In a test, compared with a situation where a catalyst is not activated, in the gas purification device arranged in this application, the filter 130 including the catalyst is disposed downstream of the ion generator 120. The ions generated by the ion generator 120 activate the catalyst, so that the temperature of the catalyst increases from a refrigerator chamber temperature of the refrigerator (a temperature from 0 to 5 degrees) to a temperature from 20 to 30 degrees, the VOC gas molecules are decomposed and purified by using the catalyst with a higher temperature, and therefore the concentration declines by 30% to 50%. Moreover, some ozone in the airflow that passes through the filter 130 is also catalyzed and decomposed, and the concentration of the ozone declines by 50%. In another test, the concentration value of the ozone in the airflow that passes through the filter 130 declines to 150 ppm or lower.

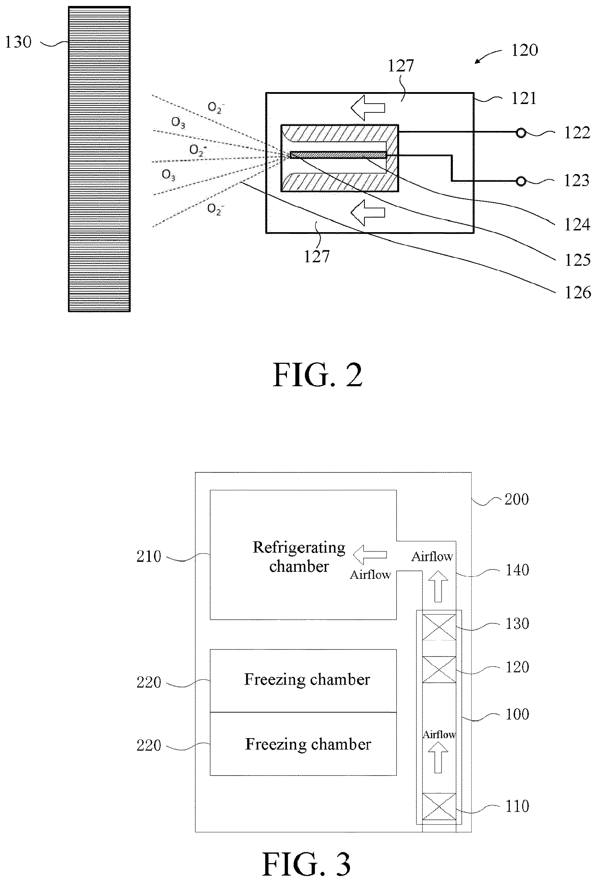

As shown in FIG. 2, the ion generator 120 includes a shell 121, a first electrode 122, and a second electrode 123. The first electrode 122 and the second electrode 123 may be a grounding electrode and a negative electrode, respectively. The negative electrode can ionize a gas, and cause negative ions, ozone, and the like (shown by 126 in the figure) which can be used for sterilization and odor removal to finally accumulate around the second electrode 123 (for example, an end 125 of the second electrode 123). An operating voltage of the negative electrode is, for example, in a range of -3 KV to -7 KV. The first electrode 122 and the second electrode 123 may alternatively be a positive electrode and a negative electrode. The positive electrode and negative electrode both can ionize a gas and cause a plasma (not shown in the figure) including positive ions, negative ions and the like to accumulate around the two electrodes.

In specific implementation, the first electrode 122 and/or the second electrode 123 may include a needle-like portion 124. Because the needle-like portion 124 is in a shape of a needle, the ions can be accumulated more effectively. Furthermore, the end 125 of the needle-like portion 124 may face the filter 130, so that ions, ozone and the like around the needle-like portion 124 are relatively closer to the filter 130, thereby helping the ions, ozone, and the like with kinetic energy to move toward the filter 130, and also helping the airflow to send the ions, ozone, and the like to the filter 130. A relative position relationship between the fan module 110 and the ion generator 120 may be adjusted so that the airflow directed by the fan module 110 flows in the ion generator 120 along an extension direction of an electrode that generates ions, so that most or all of the ions are directed to the filter 130.

The airflow may flow through the outside of the ion generator 120 to direct ions generated by the ion generator 120. In a preferred embodiment, the airflow may flow through the inside of the ion generator 120. For example, a passage 127 is disposed between the first electrode 122 and/ or the second electrode 123 and the shell 121, and the airflow (an arrow in FIG. 2 indicates a flow direction of the airflow) flows through the passage 127, thereby directing more ions, ozone, and the like.

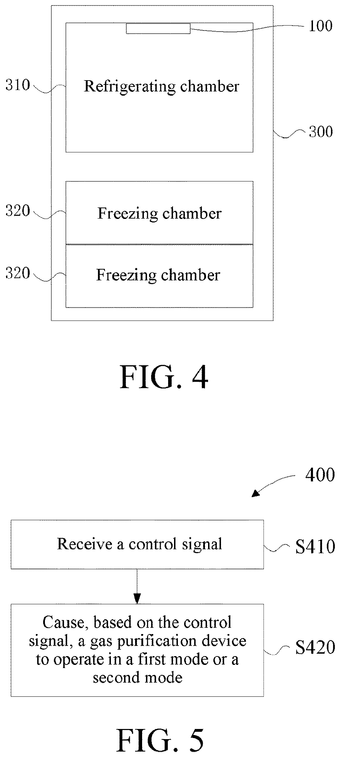

In some embodiments, as shown in FIG. 3, the gas purification device 100 is disposed in an air duct 140 that delivers cold air to a storage compartment (a refrigerating chamber 210 or a freezing chamber 220) of a refrigerator 200, that is, an airflow channel (the airflow channel is indicated by an arrow direction in the air duct 140 in FIG. 3), the fan module 110, the ion generator 120, and the filter 130 are located in the air duct 140, where the fan module 110 may be a dedicated fan used for delivering cold air to the storage compartment through the air duct 140, that is, the dedicated fan may further be used as a component of the gas purification device 100 (used for directing the airflow to the filter 130, so that odor molecules, ions, ozone, and the like in the airflow reach the filter 130). Although the air duct 140 for delivering cold air to the refrigerating chamber 210 is shown in FIG. 3, it should be understood that there is also an air duct for delivering cold air to the freezing chamber 220, and similarly, the fan module 110, the ion generator 120, and the filter 130 may further be disposed in the air duct. In some other embodiments, as shown in FIG. 1 and FIG. 4, the gas purification device 100 includes a housing 103. The airflow channel, the fan module 110, the ion generator 120, and the filter 130 are located in the housing 103. The gas purification device 100 including the housing 103 may be disposed in a refrigerator 300, for example, disposed on the top or a side of the storage compartment (a refrigerating chamber 310 or a freezing chamber 320).

The embodiments of the present invention further relate to a refrigerator. The refrigerator is a refrigeration equipment that provides a cooling space (such as a refrigerating chamber, a freezing chamber, or another storage compartment), can be used for low-temperature preservation of goods such as food, and includes a household refrigerator, an industrial refrigerator, a freezer, and the like. The refrigerator may be the refrigerator 200 shown in FIG. 3. As described above, the gas purification device 100 includes the fan module 110, the ion generator 120, and the filter 130 including the catalyst. The gas purification device 100 is disposed in the air duct 140 of the refrigerator 200. The refrigerator may alternatively be the refrigerator 300 shown in FIG. 4. As described above, the gas purification device 100 includes the housing 103. The fan module 110, the ion generator 120, and the filter 130 including the catalyst are located in the housing 103. The gas purification device 100 is disposed in the refrigerator 300 (for example, the top or a side of the storage compartment (the refrigerating chamber 310 or the freezing chamber 320)).

The embodiments of the present invention further relate to a method 400 for purification through a gas purification device 100. As described above, the gas purification device 100 includes a fan module 110, an ion generator 120, and a filter 130 including a catalyst that are disposed in an airflow channel. The filter 130 is disposed downstream of the ion generator 120, and ions generated by the ion generator 120 have kinetic energy for movement towards the filter 130. An electrode of the ion generator 120 includes a needle-like portion 124, and an end 125 of the needle-like portion 124 faces the filter 130. The gas purification device 100 is adapted to operate in a first mode or a second mode. In the first mode, the fan module 110 operates but the ion generator 120 does not operate so that an airflow passes through the filter 130, and in the second mode, the fan module 110 operates and the ion generator 120 operates so that ions included in an airflow come into contact with the catalyst when the airflow passes through the filter 130. The gas purification device 100 may be disposed in a storage compartment (for example, a refrigerating chamber or a freezing chamber) of a refrigerator, or may be disposed in an air duct 140 of the refrigerator for delivering cold air to the storage compartment.

As shown in FIG. 5, the method 400 includes step S410 (that is, receiving a control signal); and S420 (that is, causing, based on the control signal, the gas purification device 100 to operate in the first mode or the second mode).

In implementation of S410, a control signal is received. The control signal includes a control signal generated according to an operation of the refrigerator, for example, a control signal generated according to opening or closing a refrigerator door, a control signal generated according to refrigeration information of a refrigerating system, and a control signal generated according to user inputted information. The control signal further includes a control signal generated according to a concentration value of odor gas (for example, VOC) sensed by an odor sensor in the gas purification device 100.

In implementation of S420, based on the control signal, the gas purification device 100 is controlled to operate in the first mode or the second mode. In specific implementation, the gas purification device 100 may operate in the first mode based on the control signal generated according to opening of the refrigerator door, operate in the second mode based on the control signal generated according to closing of the refrigerator door, operate in the first mode or the second mode based on the control signal generated according to the refrigeration information of the refrigerating system, operate in the first mode or the second mode based on the control signal generated according to the user inputted information, and operate in the first mode or the second mode based on the control signal generated according to the concentration value sensed by the odor sensor. For example, if the sensed concentration value is higher than a preset threshold, the ion generator 120 starts operating; otherwise, the ion generator 120 does not operate. The preset threshold is in a range of 500 ppm to 1500 ppm.

Although the present invention is disclosed above, the present invention is not limited thereto. A person skilled in the art may make variations and modifications without departing from the present invention. Therefore, the protection scope of the present invention should be subject to the claims.

## Claims

**1.** A gas purification device (100) for a refrigerator (200, 300), **characterized by** comprising:
an airflow channel, having an air inlet (101) and an air outlet (102);
a fan module (110), disposed in the airflow channel, and adapted to direct an airflow from the air inlet (101) to the air outlet (102);
an ion generator (120), disposed in the airflow channel, and adapted to generate ions; and
a filter (130) comprising a catalyst, disposed in the airflow channel and downstream of the ion generator (120), where an airflow comprising the ions flows through the filter (130).

**2.** The gas purification device (100) according to claim 1, **characterized in that**, an electrode (122, 123) of the ion generator (120) comprises a needle-like portion (124), and an end (125) of the needle-like portion (124) faces the filter (130).

**3.** The gas purification device (100) according to claim 1 or 2, **characterized by** comprising a first electrode (122) and a second electrode (123), wherein the airflow flows along an extension direction of the first electrode (122) and/or the second electrode (123) in the ion generator (120).

**4.** The gas purification device (100) according to claim 3, **characterized in that**, the ion generator (120) comprises a passage (127) located between the first electrode (122) and/or the second electrode (123) and a shell (121) of the ion generator (120), and the airflow flows through the passage (127).

**5.** The gas purification device (100) according to any one of the preceding claims, **characterized in that**, the catalyst comprises a metal oxide or a precious metal.

**6.** The gas purification device (100) according to any one of the preceding claims, **characterized in that**, the ion generator (120) is adapted to continuously operate for at least 5 minutes and/or **in that**, an operating voltage of the ion generator (120) is in a range of -3 KV to -7 KV.

**7.** The gas purification device (100) according to any one of the preceding claims, **characterized in that**, a distance between the ion generator (120) and the filter (130) is in a range of 10 mm to 500 mm, in particular in a range of 20 mm to 30 mm.

**8.** The gas purification device (100) according to any one of the preceding claims, **characterized by** comprising a housing (103), wherein the airflow channel, the fan module (110), the ion generator (120), and the filter (130) are all located in the housing (103).

**9.** The gas purification device (100) according to any one of the preceding claims, **characterized in that**, the gas purification device (100) is adapted to operate in a first mode or a second mode, wherein in the first mode, the fan module (110) operates but the ion generator (120) does not operate, and in the second mode, the fan module (110) operates and the ion generator (120) operates.

**10.** The gas purification device (100) according to claim 9, **characterized in that**, the ion generator (120) is adapted to start or stop operating according to an operation of the refrigerator (200, 300).

**11.** The gas purification device (100) according to claim 9 or 10, **characterized by** comprising an odor sensor, wherein the ion generator (120) is adapted to determine, according to a concentration value of an odor gas sensed by the odor sensor, whether to operate, wherein preferably the gas purification device is adapted to determine whether the concentration value is higher than a preset threshold; if yes, the ion generator (120) operates; otherwise, the ion generator (120) does not operate.

**1812.** The gas purification device (100) according to any one of the preceding claims, **characterized in that**, the fan module (110), the ion generator (120), and the filter (130) are located in an air duct (140) adapted to deliver cold air to a storage compartment (210, 220, 310, 320) of the refrigerator (200).

**13.** A refrigerator (200, 300), **characterized by** further comprising the gas purification device (100) according to any one of claims 1 to 12.

**14.** A method for purification through a gas purification device (100), the gas purification device (100) comprising a fan module (110), an ion generator (120), and a filter (130) comprising a catalyst that are disposed in an airflow channel, the filter (130) being disposed downstream of the ion generator (120), the gas purification device (100) being adapted to operate in a first mode or a second mode, wherein in the first mode, the fan module (110) operates but the ion generator (120) does not operate so that an airflow passes through the filter (130), and in the second mode, the fan module (110) operates and the ion generator (120) operates so that ions comprised in an airflow come into contact with the catalyst when the airflow passes through the filter (130); the method comprises:
receiving a control signal; and
causing, based on the control signal, the gas purification device (100) to operate in the first mode or the second mode.

**15.** The method according to claim 14, **characterized in that**, the receiving a control signal comprises receiving a control signal generated according to an operation of a refrigerator (200, 300) and/or **in that**, the gas purification device (100) comprises an odor sensor adapted to sense a concentration value of an odor gas, and the receiving a control signal comprises receiving a control signal generated according to the concentration value.
